# EUROPÄISCHE PATENTSCHRIFT

(11) **EP 1 054 682 B1**
(45) Veröffentlichungstag und Bekanntmachung des Hinweises auf die Patenterteilung: **29.08.2001**
(21) Anmeldenummer: 99932474.2
(22) Anmeldetag: 04.02.1999
(51) Int. Cl.: A61K 35/78, A61K 31/12, A61P 25/28

(54) **VERWENDUNG VON HYPERFORIN UND HYPERFORINHALTIGEN EXTRAKTEN ZUR BEHANDLUNG UND PROPHYLAXE VON DEMENZERKRANKUNGEN**
USE OF HYPERFORIN AND HYPERFORIN-CONTAINING EXTRACTS IN THE TREATMENT AND PROPHYLAXIS OF DEMENTING DISEASES
UTILISATION DE L'HYPERFORINE ET D'EXTRAITS EN CONTENANT POUR LE TRAITEMENT ET LA PROPHYLAXIE DES MALADIES LIEES A LA DEMENCE

(30) Priorität: 13.02.1998 DE 19805946
(43) Veröffentlichungstag der Anmeldung: 29.11.2000
(73) Patentinhaber: Dr. Willmar Schwabe GmbH & Co., 76227 Karlsruhe (DE)
(72) Erfinder: CHATTERJEE, Shyam, Sunder, D-76139 Karlsruhe (DE); ERDELMEIER, Clemens, D-76139 Karlsruhe (DE); NÖLDNER, Michael, D-76139 Karlsruhe (DE)
(74) Vertreter: Bunke, Holger, Dr.rer.nat. Dipl.-Chem.
(86) Internationale Anmeldenummer: EP9900730
(87) Internationale Veröffentlichungsnummer: WO9940905

(56) Entgegenhaltungen:
- EP-A- 0 599 307
- DE-C- 19 619 512
- JOHNSON, D. ET AL: "Effects of Hypericum extract LI 160 compared with Maprotiline on resting EEG and evoked potentials in 24 volunteers" JOURNALOF GERIATRIC PSYCHIATRY AND NEUROLOGY, Bd. 7, Nr. suppl. 1, Oktober 1994 (1994-10), Seiten s44-s46, XP002110913
- LEHRL, S. ET AL: "Psychometrische Messung der Leistungskapazität unter antidepressiver Therapie mit Johanniskraut-Extrakt" NERVENHEILKUNDE, Bd. 10, 1991, Seiten 313-315, XP002110914
- SCHULZ, V. ET AL: "Klinische Studien mit Psycho-Phytopharmaka" ZEITSCHRIFT FÜR PHYTOTHERAPIE, Bd. 18, Nr. 3, 15. Juni 1997 (1997-06-15), Seiten 141-154, XP002110915
- HERBERHOLD, C.: "Pflanzliche Arzneien bessern den Score im Hirnleistungstest" FORTSCHRITTE DER MEDIZIN, Bd. 111, Nr. 6, 28. Februar 1993 (1993-02-28), Seite 50 XP002110916
- FROHLICH, W.: "Nootropika: BGA verlangt Therapie-Verbund" FORTSCHRITTE DER MEDIZIN, Bd. 112, Nr. 4, 10. Februar 1994 (1994-02-10), Seite 48 XP002110917
- LINDE, K. ET AL: "St. John's Wort for depression-an overview and meta-analysis of randomised clinical trials" BRITISH MEDICAL JOURNAL, Bd. 313, 1996, Seiten 253-258, XP002110918 in der Anmeldung erwähnt
- DATABASE WPI Week 9237 Derwent Publications Ltd., London, GB; AN 92-304661 XP002110919 & JP 04 210642 A (KAO CORP.), 31. Juli 1992 (1992-07-31)

## Beschreibung

Die Erfindung betrifft die Verwendung von Hyperforin und hyperforinhaltigen Extrakten aus Hypericum perforatum L. (Johanniskraut) zur Herstellung eines Arzneimittels zur Behandlung und Prophylaxe von Demenzerkrankungen wie zum Beispiel der Alzheimer-Krankheit.

Das Phloroglucinderivat Hyperforin der folgenden Strukturformel 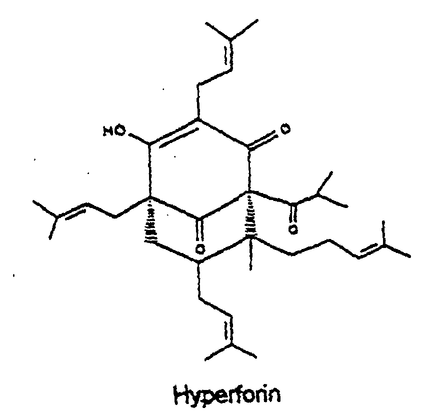 ist einer der Hauptinhaltstoffe des Johanniskrauts. Da die Substanz chemisch instabil ist, nimmt ihr Gehalt schon bei der Trocknung der Frischpflanze ab. Bei schneller Trocknung und unter schonenden Bedingungen findet man Hyperforin in der Johanniskraut-Droge noch in hoher Konzentration, die dem Gehalt frischer Pflanzen vergleichbar ist.

Hypericum-Extrakte werden seit vielen Jahren zur Behandlung von Depressionen und psychovegetativen Störungen verwendet (vgl. Linde et al., BMJ, Vol. 313, S. 253-258, (1996)). In der DE-PS 196 19 512 ist ein Verfahren zur Herstellung von Hypericum-Extrakten beschrieben, die einen stabilen Hyperforingehalt von mindestens 2 % enthalten.

Mit Hypericum-Extrakten durchgeführte pharmakologische Studien wurden beschrieben von S. Lehrl et al. in Nervenheilkunde, Band 10, 1991, Seiten 313-315, sowie von D. Johnson et al., in Journal of Geriatric Psychiatry and Neurology, Vol. 7, suppl. 1, Oktober 1994, Seiten S44-S46. Beide Studien wurden mit einem unter der Marke "Jarsin" im Handel erhältlichen Johanniskraut-Extrakt mit der Firmenbezeichnung "LI 160" durchgeführt. Welche Inhaltsstoffe der verwendete Hypericum-Extrakt "LI 160" enthält oder zum damaligen Zeitpunkt enthalten hat, ist in beiden Druckschriften nicht angegeben. Der verwendete Extrakt führte zur Verbesserung der Informationsverarbeitung bei depressiven Patienten bzw. zur Verbesserung der kognitiven Fähigkeiten der Patienten.

Aus EP-A-0 599 307 sind Hypericum-Trockenextrakte und Verfahren zu ihrer Herstellung bekannt, die arm an Hypericin sind, aber einen höheren Gehalt an Hyperforin haben als das pflanzliche Ausgangsmaterial. Diese Extrakte besitzen eine stark Serotonin-antagonistische Wirkung, und sie werden zur Herstellung psychovegetativ und antidepressiv wirksamer Arzneimittel verwendet. Diese Extrakte werden zur Behandlung von Angsterkrankungen, nervöser Agitiertheit, Migräne, gastrointestinalen Störungen und Erbrechen verwendet. Darüber hinaus sind für verschiedene Zubereitungen, die einen Hypericum-Extrakt allein oder in Kombination mit anderen Stoffen enthalten, eine Vielzahl von Anwendungen im medizinischen und kosmetischen Bereich beschrieben. Bekannt sind insbesondere auch antivirale und antibakterielle Effekte. So ist aus WO 97/22354 die Verwendung von Hypericum-Extrakten zur Behandlung viraler Erkrankungen, insbesondere von HIV, bekannt, während in der russischen Patentschrift RU-C1-2 031 645 antibakterielle Effekte und die Anwendung von Hypericum-Extrakt enthaltenden Salben zur Verhinderung von beim Geschlechtsverkehr übertragbaren Krankheiten beschrieben sind.

In tierexperimentellen Untersuchungen wurde nun überraschenderweise eine signifikante Beeinflussung kognitiver Funktionen durch Hyperforin und hyperforinhaltige Extrakte aus Hypericum perforatum L. gefunden. Eine derartige Wirkung ist bisher weder für Hyperforin noch für hyperforinhaltige Extrakte beschrieben worden und war aufgrund der bisher bekannten pharmakologischen und klinischen Effekte auch nicht zu erwarten. Im Rahmen der vorliegenden Erfindung wurde erstmalig eine Beeinflussung der Gedächtnisleistung durch einen hyperforinreichen Hypericum-Extrakt und durch Hyperforin selbst gefunden. Hyperforin und hyperforinhaltige Extrakte können somit für die Therapie von neurologischen Erkrankungen, die mit Demenz einhergehen, eingesetzt werden.

Unter Demenz versteht man eine entscheidende Abnahme der intellektuellen Leistungsfähigkeit, die als Abnahme des Gedächtnisses und des Denkvermögens in Erscheinung tritt und mit einer Beeinträchtigung der persönlichen Aktivitäten des täglichen Lebens verbunden ist. Große Autopsie-Statistiken und klinisch-epidemiologische Untersuchungen zeigen, daß neurodegenerative Erkrankungen mit ca. 60% die häufigste Ursache einer Demenz sind. Danach folgen mit ca. 20% der Fälle die sogenannten vaskulären Demenzformen und mit weiteren ca. 20% andere Ursachen (vgl. Rösler et. al., Fortschr. Med. 114, S. 351-356 (1996)). Bis heute existiert keine allgemein anerkannte Therapiemethode für demenzielle Erkrankungen, so daß sich die Behandlung auf die Verbesserung der klinischen Symptome beschränken muß.

Die Alzheimer-Demenz oder Alzheimer-Krankheit ist eine schleichend beginnende Krankheit, die gekennzeichnet ist durch anfängliche Vergeßlichkeit, zunehmende Gedächtnisstörungen und Einbußen weiterer kognitiver Fähigkeiten. Sie endet mit dem völligen geistigen Verfall und Persönlichkeitsverlust der Patienten. Eine befriedigende kausalorientierte Therapie dieser Krankheit steht bisher nicht zur Verfügung (vgl. K. Mendla, "Die Alzheimer-Krankheit: Neue Ansätze in der Pharmakotherapie", Pharm. Zeitung 141, S. 351-356 (1996)). Behandelt wird die Alzheimer-Demenz mit Acetylcholinesterase-Hemmstoffen, um die Menge des im Gehirn verfügbaren Acetylcholins zu erhöhen. Diese Behandlung führt zu einer Reihe von unerwünschten Nebenwirkungen, die keine dauerhafte Therapie zulassen (vgl. Shvaloff et al., Psychopharmacology Bulletin, Vol. 32, S. 343-352 (1996)).

Der Erfindung liegt deshalb die Aufgabe zugrunde, antidementiell wirkende Substanzen und solche Substanzen enthaltende Zusammensetzungen bereitzustellen, die weniger Nebenwirkungen haben als die bisher für die diese Indikation verwendeten Arzneimittel.

Gegenstand der Erfindung ist somit die Verwendung von Hyperforin und hyperforinhaltigen Extrakten des Johanniskrauts zur Herstellung eines Arzneimittels zur Behandlung und Prophylaxe von Demenzerkrankungen, insbesondere der Alzheimer-Krankheit, der vaskulären Demenz und von Mischformen der Demenz. Vorzugsweise enthält des Extrakt mindestens 2 Gew.-% Hyperforin. Das Hyperforin ist vorzugsweise durch Zugabe eines Stabilisators stabilisiert.

Ohne an eine bestimmte Theorie gebunden werden zu wollen, scheint die Verwendung von Hyperforin und hyperforinhaltigen Extrakten einen kausaltherapeutischen Ansatzpunkt aufzuweisen, weil nämlich überraschenderweise gefunden wurde, daß Hyperforin und die genannten Extrakte potente Stimulatoren der Proteinkinase Cγ sind. Diese Proteinkinase Cγ aktiviert die α-Sekretase, welche wiederum die Entstehung des pathogenen Amyloids Aβ verhindert. Ein besonderer Vorteil von Hyperforin und hyperforinhaltigen Extrakten liegt somit darin, daß diese nicht nur gezielt die Bildung von Amyloid Aβ verhindern, sondern auch die bei Demenzerkrankungen, insbesondere Alzheimer, häufig auftretenden psychiatrischen Begleitsymptome wie Ängstlichkeit, Depressionen und andere psychovegetative Störungen wirksam bekämpfen können.

Ein erfinderungsgemäß verwendbarer hyperforinhaltiger Extrakt wurde wie folgt gewonnen:

30 kg getrocknete, gemahlene Johanniskraut-Droge wurden in eine Hochdruckextraktionsanlage gegeben und bei 280 x 10⁵ Pa (280 bar) und 40 °C mit Kohlendioxid extrahiert. Dabei wurden 30 kg CO₂ je Kilogramm Droge eingesetzt. Nach der Extraktion wurde der Druck auf 60 x 10⁵ Pa (60 bar) reduziert, wobei der Extrakt abgeschieden wurde. Der Extrakt wurde der Apparatur entnommen und durch Erhitzen auf ca. 45 °C von mitextrahiertem Wasser abgetrennt. Man erhielt 1,25 kg Extrakt mit einem Hyperforin-Gehalt von 34,5%. Zur Stabilisierung des Hyperforins wurden dem warmen Extrakt 500 mg Ascorbinsäurestearat zugegeben.

Zur Isolierung von Hyperforin aus dem so gewonnenen Extrakt wurde wie folgt vorgegangen:

31 g des nach vorstehend beschriebenem Verfahren erhaltenen Extraktes wurden unter Rühren, Stickstoffatmosphäre und Lichtschutz in 3 Liter n-Heptan, das mit Methanol gesättigt war, gelöst. Die erhaltene Extraktlösung wurde dann einmal mit 1,5 1 Methanol, das mit n-Heptan gesättigt war, und dreimal mit je einem Liter des gleichen Lösungsmittels ausgeschüttelt. Die Methanolphasen wurden vereinigt und bei einer Wasserbadtemperatur von maximal 45 °C am Rotationsverdampfer zur Trockne gebracht. Im Vakuumtrockenschrank wurde über Nacht bei Raumtemperatur nachgetrocknet. Man erhielt so 20,14 g Methanolphase.

Die Gesamtmenge an Methanolphase wurde in 100 ml Methanol gelöst. Die so erhaltene Stammlösung wurde zur präparativen Hochdruck-Flüssigchromatographie (HPLC) eingesetzt: Sie wurde ca. 2 Minuten im Ultraschallbad entgast, in einem Braunglas unter Stickstoffatmosphäre bei - 18 °C gelagert und der präparativen HPLC unterworfen. Von der Stammlösung wurden für jede präparative HPLC-Trennung 3,7 ml verwendet, was einer Substanzmenge von 745 mg entspricht. Die Bedingungen der präparativen HPLC waren wie folgt:

| | |
|---|---|
| Eluens | 88 Vol.-% Methanol, reinst |
| Detektion | 270 nm |
| Fluß | 90 ml/min. |
| Säule | Eurospher 100-C18, 7 µm, ID 32 mm. |

Der Hyperforin-Peak wurde von jeder einzelnen präparativen Trennung gesammelt. Die Hyperforin enthaltenden HPLC-Eluate wurden zunächst bei - 18 °C gelagert. Dann wurden sie vereinigt und zügig am Rotationsverdampfer bei maximal 45 °C Wasserbadtemperatur, unter Lichtschutz und Stickstoffbegasung des Vorratsgefäßes wäßrig aufkonzentriert. Diese Konzentrate wurden mit Hilfe von Methanol in Braungläser umgespült und bis zur endgültigen Vereinigung bei - 18 °C unter Stickstoffatmosphäre gelagert. Nach dem Abziehen des Methanols und einer Hochvakuumtrocknung wurden 9,4 g Hyperforin erhalten, welches durch UV-, IR- und ¹H-NMR- und ¹³C-NMR-Spektren charakterisiert wurde.

Als Tiermodell zum Nachweis der kognitiven Wirkung des Hyperforins und des nach dem oben beschriebenen Verfahren gewonnenen hyperforinhaltigen Extrakts wurde das sogenannte "conditioned avoidance"-Modell gewählt.

Bei diesem Modell wird ein geschlossener Kunststoffkäfig, die "conditioned avoidance box", benutzt, der in der Mitte durch eine Trennwand in zwei gleich große Kammern oder Kompartimente geteilt ist. In der Mitte der Trennwand befindet sich eine Tür, die durch eine automatische Steuerung geöffnet und geschlossen werden kann. Der Boden beider Kammern besteht aus einem elektrisch leitenden Gitterrost. An den Wänden jeder Kammer befindet sich eine Lampe und ein Lautsprecher, über die Licht- und Tonsignale abgegeben werden können.

Eine einzelne Ratte wird zum Training jeweils in das rechte Kompartiment der Box gesetzt, wobei die Tür der Trennwand geschlossen bleibt. Nachdem sich die Ratte 10 Minuten an die neue Umgebung gewöhnt hat, beginnt ein automatisiertes Programm mit dem Öffnen der Tür. Nach 20 Sekunden erscheint für 3 Sekunden ein kombiniertes Ton-Licht-Signal in der Kammer, in der sich die Ratte aufhält (conditioned stimulus). Bleibt die Ratte trotz des Stimulus in der Kammer, folgt ein 3 Sekunden währender Stromreiz über die Gitterstäbe (unconditioned stimulus). Anschließend beginnt ein neuer Zyklus. Wechselt die Ratte vor oder während des conditioned stimulus in die gegenüberliegende Kammer, vermeidet sie den Stromreiz, und der neue Zyklus beginnt in der anderen Kammer. Insgesamt muß die Ratte 40 derartige Durchgänge am Tag absolvieren. Diese Prozedur wiederholt sich an sieben aufeinander folgenden Tagen in gleicher Art und Weise. Anschließend folgt eine Pause von 10 Tagen, in denen weder behandelt noch trainiert wird. Am 17. Versuchstag werden die Tiere ein letztes Mal in die Box gesetzt, jedoch mit dem Unterschied, daß keine Stromreize mehr appliziert werden.

Durch das tägliche Trainingsprogramm erlernen die Ratten, daß sie den Stromreiz durch Wechseln der Kompartimente vermeiden können (Vermeidungsverhalten). Substanzen, die die Lern- oder Gedächtnisfunktionen verbessern, führen zu einem schnelleren Erlernen des Vermeidungsverhaltens und dadurch zu einer geringeren Anzahl von Elektroschocks.

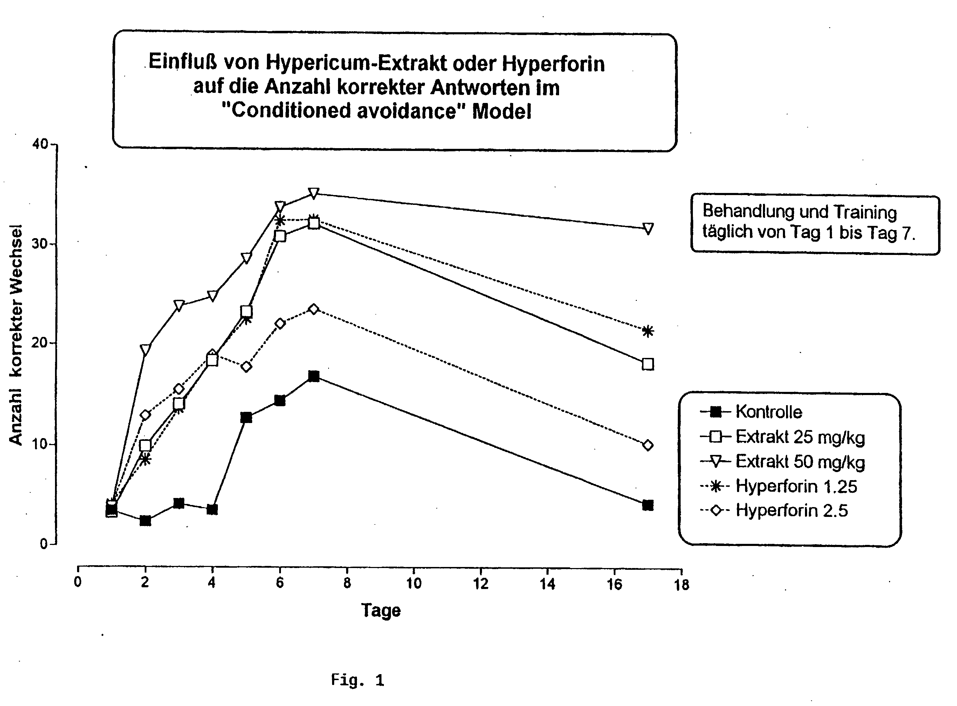

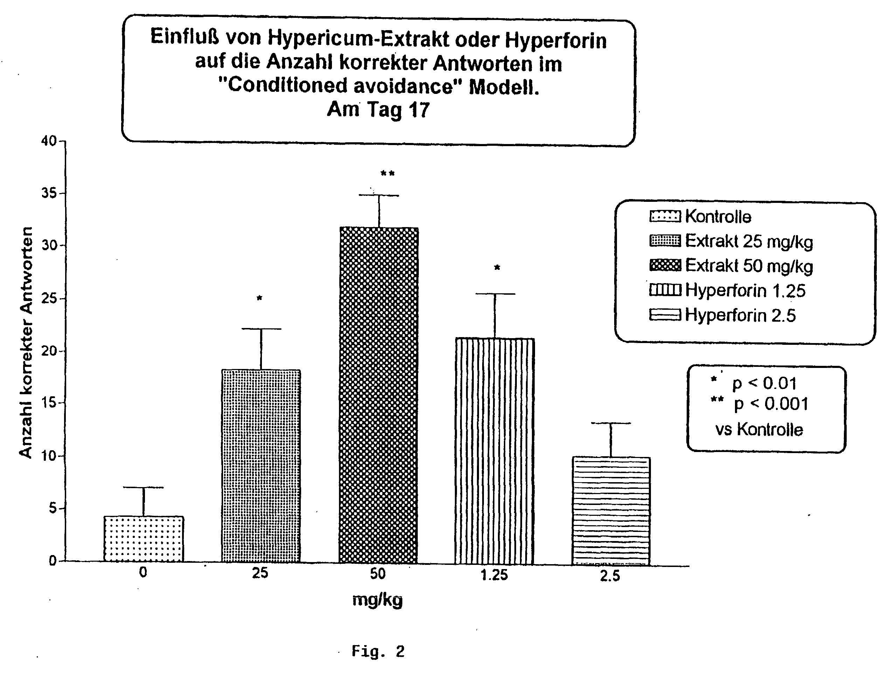

Tägliche orale Applikationen von 25 oder 50 mg/kg Hypericum-Extrakt sowie von 2,5 oder 5 mg/kg reinem Hyperforin führen zu einem deutlich schnelleren Erlernen des Vermeidungsverhaltens. In der beschriebenen Versuchsanordnung zeigt sich dieser Effekt in einer wesentlich höheren Anzahl korrekter Vermeidungsreaktionen während der siebentägigen Behandlungs- und Trainingsphase (vgl. Fig. 1). Das erlernte Verhalten wird von den mit Hypericum-Extrakt oder mit Hyperforin behandelten Tieren weniger schnell vergessen, was an der Reaktion der Tiere nach der zehntägigen Behandlungs- und Trainingspause am 17. Tag des Versuchs erkennbar wird (vgl. Fig. 2).

Die in Fig. 1 wiedergegebenen Graphen geben die Anzahl korrekter Wechsel der Versuchstiere von einem Kompartiment in das andere vom 1. bis zum 7. Tag und- nach zehntägiger Pause - am 17. Tag wieder, und zwar sowohl für unbehandelte Versuchstiere als auch für Versuchstiere, die täglich 25 mg/kg bzw. 50 mg/kg Hypericum-Extrakt p.o. verabreicht bekommen haben, und solche Versuchstiere, die täglich 1,25 bzw. 2,5 mg/kg reines Hyperforin p.o. verabreicht bekommen haben. Dabei fällt auf, daß reines Hyperforin weniger wirksam ist als der Hypericum-Extrakt in einer Dosis von 50 mg/kg.

In Fig. 2 ist die Anzahl der korrekten Antworten der Versuchstiere am 17. Tag noch einmal in anderer graphischer Form dargestellt.

## Patentansprüche

1. Verwendung von Hyperforin und hyperforinhaltigen Extrakten aus Hypericum perforatum L. (Johanniskraut) zur Herstellung eines Arzneimittels zur Behandlung und Prophylaxe von Demenzerkrankungen, insbesondere der Alzheimer-Krankheit, der vaskulären Demenz und von Mischformen der Demenz.

2. Verwendung nach Anspruch 1, **dadurch gekennzeichnet**, daß der Extrakt mindestens 2 Gew.-% Hyperforin enthält.

3. Verwendung nach Anspruch 1 oder 2, **dadurch gekennzeichnet**, daß das Hyperform durch Zugabe eines Stabilisators stabilisiert ist.

## Claims

1. Use of hyperforin and hyperforin-containing extracts of *Hypericum perforatum L.* (St. John's wort) in the preparation of a medicament for the treatment and prophylaxis of dementia diseases, in particular Alzheimer's disease, vascular dementia and mixed forms of dementia.

2. Use according to claim 1, **characterized in that** the extract contains at least 2% by weight of hyperforin.

3. Use according to claim 1 or claim 2, **characterized in that** the hyperforin is stabilized by the addition of a stabilizer.

## Revendications

1. Utilisation d'hyperforine et d'extraits contenant de l'hyperforine, de l'*Hypericum perforatum L*. (millepertuis), pour la préparation d'un médicament pour le traitement et la prophylaxie de maladies démentielles, en particulier de la maladie d'Alzheimer, de la démence vasculaire et de formes mixtes de démence.

2. Utilisation selon la revendication 1, **caractérisée en ce que** l'extrait contient au moins 2 pour cent en poids d'hyperforine.

3. Utilisation selon la revendication 1 ou 2, **caractérisée en ce que** l'hyperforine est stabilisée par l'addition d'un stabilisateur.
